# FASCICULE DE BREVET EUROPEEN

(11) **EP 0 547 930 B1**
(45) Date de publication et mention de la délivrance du brevet: **16.07.1997**
(21) Numéro de dépôt: 92403206.3
(22) Date de dépôt: 27.11.1992
(51) Int. Cl.: C07B 39/00, C07C 17/00

(54) **Procédé de fluoration en phase liquide**
Fluorierungsverfahren in der Flüssigphase
Liquid-phase fluorination process

(30) Priorité: 17.12.1991 FR 9115630
(43) Date de publication de la demande: 23.06.1993
(73) Titulaire: ELF ATOCHEM S.A., 92800 Puteaux (FR)
(72) Inventeur: Berthe, Bernard, F-13011 Marseille (FR)
(74) Mandataire: Hirsch, Marc-Roger

(56) Documents cités:
- FR-A- 2 388 785
- FR-A- 2 652 573
- US-A- 2 005 709

## Description

La présente invention a trait à un procédé amélioré de fluoration en phase liquide en présence d'un catalyseur. Elle concerne les produits organiques fluorés résultant de ce procédé.

Des procédés de fluoration en phase liquide, en présence d'un catalyseur, basés sur la réaction de l'acide fluorhydrique et d'une matière première organique chlorée sont connus depuis longtemps. Ils sont en général mis en oeuvre dans une installation comprenant un réacteur, une colonne de séparation et un condenseur. La matière première organique chlorée et l'acide fluorhydrique sont alimentés en continu au réacteur et les produits de la réaction, à savoir le produit organique fluoré ou chlorofluoré et l'acide chlorhydrique formés sont soutirés de l'installation en aval du condenseur.

FR-A-2 652 573 divulgue un procédé et un dispositif de fluoration, ledit dispositif comprenant un réacteur alimenté en réactifs, surmonté d'une colonne et d'un condenseur de rétrogradation. Un soutirage est prévu au niveau du réacteur lui-même.

A titre de produits organiques chlorofluorés particulièrement concernés par la présente invention, on citera, entre autres, à titre d'exemples, sans que cela ne puisse constituer une quelconque limitation de l'invention:
- CFCl₃: le fluorotrichlorométhane (F11)
- CF₂Cl₂: le difluorodichlorométhane (F12)
- CHClF₂: le chlorodifluorométhane (F22)
- CFCl₂-CF₂Cl: le trichloro-1,1,2-trifluoro-1,2,2-éthane (F113)
- CF₂Cl-CH₂Cl: le dichloro-1,2-difluoro-1,1-éthane (F132b)
- CF₃-CH₂Cl: le chloro-1-trifluoro-2,2,2-éthane (F133a)
- CF₂Cl-CH₃: le chloro-1-difluoro-1,1-éthane (F142b)
- CFCl₂-CH₃: le dichloro-1,1-fluoro-1-éthane (F141b)

Bien entendu, les matières premières, les rapports molaires des réactifs, le catalyseur, la proportion de catalyseur ainsi que la température et la pression de mise en oeuvre de la réaction, entre autres, seront choisis en fonction des produits de fluoration que l'on souhaite obtenir. Les catalyseurs employés sont dans ce type de réactions, en général, des halogénures des éléments des groupes IVa, IVb, Va, Vb, VIa, VIb et VIII de la Classification Périodique des Eléments.

Sont particulièrement utilisés à cette fin les halogénures d'antimoine pentavalent, plus particulièrement le pentachlorure d'antimoine. Leur activité est maintenue par addition permanente d'halogène, en particulier de chlore. L'halogène et le catalyseur forment le système catalytique.

Il est cependant à remarquer que ces réactions de fluoration sont en général accompagnées de réactions parasites telles que déshydrochloration, dimérisation, trimérisation, etc. suivies de fluorations et/ou de chlorations. De plus, des sous-produits générés par les impuretés éventuellement présentes dans les matières premières organiques peuvent également être formés. Ces sous-produits, qu'ils résultent de réactions parasites ou de réactions avec les impuretés présentes, sont dans certains cas plus lourds, selon le critère de leur tension de vapeur, que les produits de fluoration que l'on cherche à obtenir.

Du fait de la conception de l'appareillage, sauf action particulière, une partie au moins des sous-produits formés s'accumule dans le réacteur et conduit soit à une perte de sélectivité par action sur les équilibres liquide-vapeur, soit à une désactivation du catalyseur.

Face à cette génération de sous-produits et à la nécessité de maîtriser la composition du milieu réactionnel, deux techniques ont jusqu'à présent été utilisées:

La première consiste à utiliser les conditions de fluoration drastiques de manière à éliminer autant que possible ces sous-produits en les fluorant et en les "purgeant" par le flux des produits principaux en tête de colonne de rétrogradation. C'est notamment le cas des procédés bien connus de production des dérivés F11, F12, F22 et F113, conduits en général en milieu catalytique concentré.
La seconde consiste à éliminer ces sous-produits par une purge effectuée sur le réacteur. C'est notamment le cas des procédés conduits en milieu catalytique dilué dont on peut citer deux exemples: celui de la production de F142b (cf. brevet n° FR 2 652 573) et celui de la production de F133a décrite dans l'exemple 1.

Pour des raisons diverses, notamment de sélectivité, la première technique consistant à contrôler autant que possible la composition du milieu réactionnel par des conditions de fluoration poussée n'est pas toujours applicable.

Par ailleurs, la seconde technique de contrôle de la composition du milieu réactionnel par soutirage du réacteur présente l'inconvénient de créer un flux important de catalyseur qu'il est nécessaire de traiter pour son élimination ou son recyclage.

La présente invention se propose de pallier les inconvénients des procédés connus par contrôle de la composition du milieu réactionnel lors d'une fluoration en phase liquide menée en présence d'un catalyseur. En fait, le procédé selon l'invention permet de contrôler avec plus grande précision la composition de la masse réactionnelle notamment eu égard aux proportions de catalyseur présentes, donc d'obtenir une constance de la qualité du produit fluoré préparé. De plus, il permet de réduire fortement les pertes possibles en catalyseur ou les coûts de traitement des sous-produits formés en vue de la récupération du catalyseur que leur soutirage entraîne.

La présente invention a en fait pour objet un procédé tel que défini dans la revendication 1.

Cette technique nouvelle présente l'avantage de diminuer considérablement le flux de catalyseur à traiter. Les catalyseurs utilisés, en général les chlorofluorures d'antimoine, sont en effet plus lourds que les produits organiques fluorés recherchés et le soutirage intermédiaire précité conduit donc à un effluent liquide nettement moins concentré en catalyseur.

Quoiqu'il puisse sembler évident à l'homme de l'art que, en procédant à ce soutirage intermédiaire, on entraînait moins de catalyseur qu'en procédant au soutirage au pied du réacteur, l'homme de l'art était en fait dissuadé d'employer une telle technique car elle lui semblait comporter l'inconvénient, comparé à la technique de purge au pied du réacteur, de conduire de façon inhérente à une accumulation de sous-produits lourds en cuve du réacteur qui obérerait le déroulement du procédé. Cependant, de façon surprenante et totalement inattendue, ceci n'est pas le cas et ceci conduit à la possibilité de poursuivre le déroulement du procédé sur des périodes de très longues durées avec d'excellents rendements.

Selon un mode de réalisation de la présente invention, le soutirage intermédiaire est effectué sur la fraction lourde quittant la zone de séparation.

Selon un autre mode de réalisation de la présente invention, le soutirage intermédiaire est effectué dans la zone inférieure de la zone de séparation sur une fraction lourde légèrement différente de la fraction lourde retirée en pied de la zone de séparation et provenant d'un étage théorique supérieur à celui correspondant au niveau du retrait en pied et inférieur au niveau d'alimentation en mélange réactionnel de ladite zone de séparation.
Bien entendu, la fraction lourde recueillie en pied de la zone de séparation peut être aussi bien une phase liquide qu'une phase gazeuse. Dans un mode de mise en oeuvre de la présente invention, donné à titre d'exemple ci-dessous, elle consiste de préférence en une phase liquide.

Le choix des conditions de fonctionnement: température du réacteur, pression, teneur en catalyseur, débits d'alimentation des matières premières est adapté de façon à obtenir les produits de fluoration recherchés en aval du condenseur.

Le débit de soutirage intermédiaire est adapté de façon à maintenir constante, à la valeur désirée, la teneur en lourds de la masse réactionnelle. Un contrôle analytique par exemple par chromatographie, permet de suivre l'évolution de la composition réactionnelle et d'ajuster ce débit de soutirage latéral.

La présente invention a aussi pour objet une installation de fluoration en phase liquide telle que définie dans la revendication 4

Selon une forme d'exécution de la présente invention, la conduite de soutirage est placée en pied de ladite colonne de séparation.

Selon une autre forme d'exécution de la présente invention, la conduite de soutirage est placée à un niveau compris entre un plateau théorique au-dessus du niveau du pied et un plateau théorique en-dessous du niveau d'alimentation de ladite colonne de séparation. Avantageusement, la conduite qui assure le recyclage de la fraction lourde collectée en pied de la colonne de séparation vers le réacteur de fluoration est un tube plongeant.

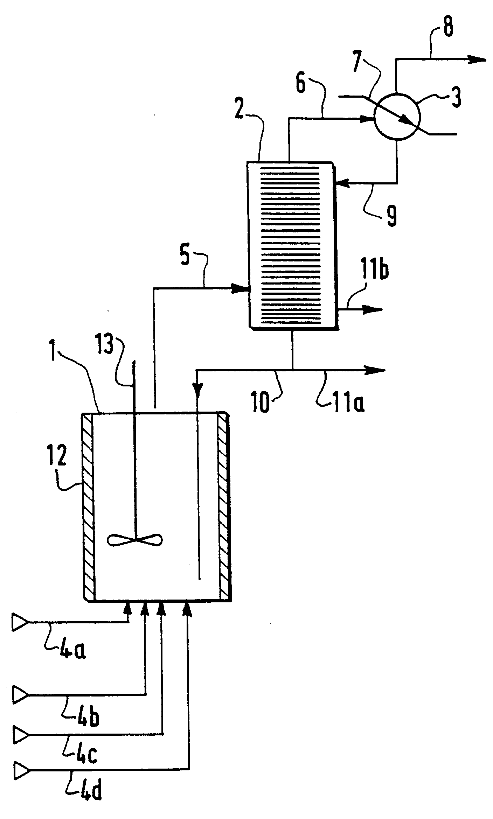

D'autres buts et avantages de la présente invention apparaîtront à la lecture de la description suivante et du schéma d'une installation susceptible de permettre la mise en oeuvre du procédé selon l'invention, donnée à titre illustratif et nullement limitatif.
La figure jointe s'applique à la fluoration du trichloréthylène en F133a par catalyse à l'aide d'antimoine, mais il est évident que ces exemples ne sont donnés qu'à titre illustratif et que la fluoration de bien d'autres produits peut être mise en oeuvre sans que l'on ne s'échappe de l'esprit de l'invention.

Cette installation comporte un réacteur 1 en inox, une colonne de séparation 2, un condenseur partiel, quasi-total ou total 3 avec une sortie gaz et une sortie liquide.

Les conduites d'amenée des réactifs sont schématisés par les conduites 4a, 4b, 4c et 4d, correspondant respectivement au trichloréthylène, c'est-à-dire la charge organique, à l'acide fluorhydrique, au chlore et au pentachlorure d'antimoine. Ces conduites peuvent, par ailleurs, être rassemblées en une conduite unique. La conduite 5 relie la tête du réacteur 1 à la colonne de séparation 2, une conduite 6 relie la tête de cette colonne de séparation 2 au condenseur 3, refroidi par un circuit 7 de fluide réfrigérant et dont une conduite 8 en tête permet la récupération du produit organique fluoré recherché et des volatils dont le chlore et l'acide fluorhydrique n'ayant pas réagi tandis que la conduite 9 relie le pied du condenseur 3 en tête de la colonne de séparation 2 assurant ainsi le reflux de celle-ci. Une conduite de collecte 10 relie le pied de la colonne de séparation 2 au fond du réacteur 1 afin d'assurer un recyclage; la conduite 10 est de préférence un tube plongeant. Le soutirage est obtenu à l'aide soit d'une conduite 11a disposée sur la conduite 10 ou à l'aide d'une conduite 11b disposée à un niveau intermédiaire sur la colonne de séparation 2, compris entre un plateau théorique au-dessus du niveau du pied et un plateau théorique en-dessous de l'alimentation par la conduite 5.

Le réacteur 1 est muni d'une double enceinte 12 qui permet de réguler la température du réacteur et d'un agitateur 13.

On soutire par la conduite 8 une fraction gazeuse contenant le produit organique fluoré résultant du procédé et les produits volatils résiduels dont le chlore et l'acide fluorhydrique n'ayant pas réagi, et on retire par la conduite 11a ou 11b un courant de la fraction lourde recueillie en pied de la colonne de séparation 2 ou à un niveau intermédiaire, contenant une partie du catalyseur entraîné.

Les fractions retirées par la conduite 8 sont dirigées vers des étapes de traitement en vue de la récupération des produits et de leur éventuel recyclage. L'adjonction de pentachlorure d'antimoine est réglée de manière à conserver une teneur en antimoine constante dans le milieu réactionnel se trouvant dans le réacteur. Le soutirage de la fraction lourde par la conduite 11a ou 11b est réglé de manière à maintenir constant le taux de produits lourds dans le milieu réactionnel.

On ajuste le débit de la conduite 9 de façon à obtenir un taux de reflux permettant de ne recueillir en phase gazeuse par la conduite 8 qu'un mélange contenant le chlore et l'acide fluorhydrique n'ayant pas réagi ainsi que l'acide chlorhydrique sous-produit et le produit organique fluoré recherché, dans ce cas le produit F133a. On recycle, vers la colonne de séparation 2, la phase liquide séparée dans le condenseur 3, contenant les produits sous-fluorés, dont notamment le F132b.

On donne ci-dessous des exemples mis en oeuvre dans une installation pilote de laboratoire comprenant un réacteur ayant une capacité de 20 litres, formé d'Inox 316L, d'une colonne de séparation, d'un condenseur et d'un séparateur de phases du type classiquement utilisé en laboratoire.

L'exemple 1 correspond au déroulement d'un procédé selon l'état de la technique, comportant un soutirage direct au pied du réacteur tandis que les exemples 2 et 3 correspondent à un déroulement du procédé conforme à l'invention avec un soutirage intermédiaire effectué sur la phase lourde, en l'espèce la phase liquide, en cuve de la colonne de séparation.

### EXEMPLE 1 - Cas du soutirage direct au pied du réacteur

Les conditions de fonctionnement de l'installation sont les suivantes:

| | |
|---|---|
| . température dans le réacteur | 130°C |
| . pression de fonctionnement | 17 bars abs. |
| . teneur en antimoine dans le réacteur | 1% en poids |
| . productivité en F133a: 1,5 moles/h/litre de réacteur | |
| . débit chlore : 2,5% molaire par rapport au trichloréthylène | |

Le débit d'acide fluorhydrique est régulé de façon à maintenir le volume réactionnel constant.

On ajuste le reflux de telle sorte que la teneur en F132b dans le distillat soit nulle.

Le débit du soutirage liquide effectué directement sur le réacteur est ajusté de façon à maintenir le taux de produits lourds dans le réacteur à 60% en poids.

Le taux de soutirage en pied du réacteur nécessaire pour maintenir cette teneur est de 0,22 kg/h. Cette purge contient 1% en poids d'antimoine. On soutire donc 2,2 g/h d'antimoine qu'il convient de compenser par une alimentation au réacteur de 5,4 g/h de SbCl₅ pour maintenir la teneur en catalyseur constante dans le réacteur.

### EXEMPLE 2 - Cas du soutirage intermédiaire sur la fraction lourde en pied de la colonne de séparation

On maintient le taux de lourds dans le réacteur à la même valeur, c'est-à-dire 60% en poids, mais effectue un soutirage intermédiaire sur la phase liquide s'échappant en pied de la colonne de séparation à l'aide de la conduite 11a.

Les conditions de la réaction et de reflux sont identiques à celles de l'exemple 1.

Le taux de soutirage nécessaire pour maintenir cette teneur est alors de 0,40 kg/h. Ce soutirage intermédiaire ne contient plus que 100 ppm d'antimoine. On soutire donc 0,04 g/h d'antimoine.

On fonctionne dans ces conditions pendant 800 heures. On suit la composition du réacteur par chromatographie. Au bout de 800 heures, le chromatogramme est identique à celui après 100 heures de fonctionnement.

Il n'apparaît aucun nouveau produit et il n'y a pas d'accumulation des plus lourds d'entre eux. Le soutirage en pied de la colonne permet donc de maintenir la composition du réacteur.

Ces deux exemples, en comparaison l'un de l'autre, mettent en évidence l'intérêt du soutirage intermédiaire en pied de la colonne de séparation par rapport à un soutirage direct en pied du réacteur pour ce qui concerne les teneurs en catalyseurs extraites.

La teneur en antimoine de la fraction extraite par soutirage effectué selon l'exemple 2 (donc en pied de la colonne) est 100 fois inférieure à celle de la fraction retirée du réacteur dans le cas du fonctionnement selon l'exemple comparatif 1.

En 100 heures, on a soutiré l'équivalent de la charge catalytique dans le réacteur par une purge directe sur le réacteur. Par contre, il faut plus de 6 000 heures de fonctionnement pour en soutirer l'équivalent par le soutirage intermédiaire en pied de la colonne (exemple 2).

### EXEMPLE 3 - Cas du soutirage en pied de la colonne de séparation (conditions de reflux différentes de l'exemple 2)

On reste dans les mêmes conditions de fonctionnement que dans l'exemple 2, mais, cependant on ajuste le débit de reflux dans la colonne de séparation au niveau du condenseur afin d'obtenir une teneur de 1% de F132b par rapport au F133a recueilli en aval de condenseur.

Le taux de soutirage intermédiaire par la conduite 11a en pied de la colonne nécessaire pour maintenir le taux de lourds dans le réacteur à 60% en poids est de 0,28 kg/h. La teneur en antimoine dans ce soutirage est de 100 ppm.

Le débit de soutirage intermédiaire nécessaire pour maintenir, dans l'exemple 2, le taux de lourds dans le réacteur est d'environ 80% supérieur à celui qui serait effectué par une purge directe en cuve du réacteur (exemple 1). Si on diminue le débit de reflux (exemple 3), alors le débit de soutirage latéral diminue et est d'environ 25% supérieur à celui de l'exemple 1.

Au niveau de la facilité de conduite de l'installation et du traitement des purges, on conçoit l'énorme avantage du soutirage intermédiaire par rapport à un soutirage direct au pied du réacteur.

### EXEMPLE 4 - Cas du soutirage intermédiaire sur une fraction lourde de la colonne de séparation à un niveau de la colonne de séparation légèrement supérieure à celui du retrait de la fraction lourde.

On opère selon les conditions décrites dans l'exemple 2, mais en procédant à un soutirage intermédiaire à un niveau de la colonne de séparation correspondant à celui d'un plateau théorique au-dessus de celui correspondant au fond de la cuve. En ce cas, la conduite de soutirage, au lieu d'être disposée sur la conduite 10, est située sur la partie inférieure de la colonne de séparation 2 à un niveau supérieur à celui auquel le prélèvement par la conduite 10 est effectué. Cette conduite est représentée par la conduite 11b. Bien entendu, alors que le soutirage dans cet exemple est prévu à un niveau correspondant à celui d'un étage théorique supérieur à celui du fond de la cuve, il peut être effectué à un niveau supérieur à celui-ci.

On obtient des résultats sensiblement identiques à ceux obtenus dans le cas de l'exemple 2 et les mêmes conclusions s'appliquent.

## Revendications

1. Procédé de fluoration en phase liquide, en présence d'un catalyseur, consistant en la réaction, dans une zone de réaction, de l'acide fluorhydrique et d'une matière première organique, en la séparation, dans une zone de séparation, du mélange réactionnel en au moins une fraction légère, contenant les produits organiques fluorés recherchés et au moins une première partie des produits organiques sous-fluorés formés, et une fraction lourde comprenant, entre autres, le reste des produits organiques sous-fluorés formés, en la condensation partielle de la fraction légère précitée en vue d'obtenir une phase gazeuse contenant les produits organiques fluorés recherchés et une phase liquide contenant la première partie des produits organiques sous-fluorés précitée, ladite fraction lourde étant renvoyée vers la zone de réaction et ladite phase liquide étant retournée à titre de reflux au sommet de la zone de séparation, ce procédé étant caractérisé en ce que ladite fraction lourde, recueillie en pied de la zone de séparation, comprenant, entre autres, le reste des produits organiques sous-fluorés formés contient, en outre, une partie du catalyseur entraîné et en ce qu'un soutirage intermédiaire est effectué en pied, ou à proximité du pied de ladite zone de séparation à un niveau correspondant au moins à un plateau théorique au-dessus du niveau du retrait en pied et au moins à un plateau théorique en-dessous du niveau d'alimentation en mélange réactionnel de ladite zone de séparation.

2. Procédé selon la revendication 1, caractérisé en ce que le soutirage intermédiaire est effectué sur la fraction lourde quittant la zone de séparation.

3. Procédé selon la revendication 1, caractérisé en ce que le soutirage intermédiaire est effectué dans la zone inférieure de la zone de séparation sur une fraction lourde légèrement différente de la fraction lourde retirée en pied de la zone de séparation et provenant d'un étage théorique supérieur à celui correspondant au niveau du retrait en pied et inférieur au niveau d'alimentation en mélange réactionnel de ladite zone de séparation.

4. Installation de fluoration en phase liquide comprenant un réacteur (1) de fluoration alimenté en acide fluorhydrique, charge organique, système catalytique; une conduite (5) en tête dudit réacteur et alimentant une colonne de séparation (2); un condenseur (3) placé en tête de ladite colonne (2) et alimenté par la conduite (6) et fournissant une fraction gazeuse qui est recueillie par la conduite (8) et une fraction liquide qui est renvoyée en tête de ladite colonne (2) par la conduite (9) afin d'assurer le reflux de celle-ci; une conduite de collecte (10) située en pied de ladite colonne (2) et collectant la fraction lourde en pied de ladite colonne (2) pour la recycler en pied dudit réacteur (1); et une conduite de soutirage (11a,11b) située sur ladite conduite de collecte (10) ou à un niveau intermédiaire compris entre un plateau théorique au-dessus du niveau du pied et un plateau théorique en-dessous du niveau d'alimentation de ladite colonne.

5. Installation de fluoration en phase liquide selon la revendication 4, dans laquelle la conduite de soutirage (11a) est placée en pied de ladite colonne de séparation (2).

6. Installation de fluoration en phase liquide selon la revendication 4, dans laquelle la conduite de soutirage (11b) est placée à un niveau compris entre un plateau théorique au-dessus du niveau du pied et un plateau théorique en-dessous du niveau d'alimentation de ladite colonne de séparation (2).

7. Installation de fluoration en phase liquide selon l'une quelconque des revendications 4 à 6, dans laquelle la conduite (10) qui assure le recyclage de la fraction lourde collectée en pied de la colonne de séparation (2) vers le réacteur (1) de fluoration est un tube plongeant.

## Claims

1. A process for carrying out liquid phase fluorination in the presence of a catalyst, consisting in reacting hydrofluoric acid and an organic starting material in a reaction zone, and in separating, in a separation zone, the reaction mixture into on the one hand at least one light fraction containing the desired fluorinated organic products and at least a first part of the sub-fluorinated organic products formed, and on the other hand a heavy fraction comprising, among other things, the remainder of the sub-fluorinated organic products formed, and consisting further in partial condensation of the said light fraction in order to obtain a gaseous phase containing the desired fluorinated organic products and a liquid phase containing said first part of the said sub-fluorinated organic products, said heavy fraction being returned to said reaction zone and said liquid phase being returned as a reflux to the top of said separation zone, wherein said heavy fraction recovered at the bottom of said separation zone and comprising, among other things, the remainder of the sub-fluorinated organic product formed furthermore contains a part of the entrained catalyst and wherein intermediate recovery is carried out at the bottom of said separation zone, or at a level in the proximity thereof corresponding at least to that of a theoretical plate situated above a withdrawal point at the bottom of said column and at least to that of a theoretical plate situated below the reaction mixture feed level of said separation zone.

2. A process according to claim 1, characterized in that said intermediate recovery is carried out on the heavy fraction leaving said separation zone.

3. A process according to claim 1, characterized in that said intermediate recovery is carried out in the lower region of said separation zone on a heavy fraction slightly different from the heavy fraction removed at the bottom of said separation zone and originating from a theoretical stage higher than a stage corresponding to removal carried out at the bottom of said column and below the reaction mixture feed level of said separation zone.

4. A liquid phase fluorination installation comprising a fluorination reactor (1) supplied with hydrofluoric acid, an organic charge, and a catalyst system; a line (5) from the head of said reactor and feeding a separation column (2); a condensor (3) linked to the head of said column (2) fed from line (6) and supplying a gaseous fraction which is recovered by a line (8), and a liquid fraction which is returned to the head of said column (2) by a line (9) in order to provide reflux thereof; a collection line (10) located at the bottom of said separation column (2) collecting the heavy fraction at the bottom of said separation column (2) for recycling thereof to the bottom portion of said reactor (1); and a recovery line (11a, 11b) located on said collection line (10) or at an intermediate level comprised between that of a theoretical plate above the level of the bottom of said column and that of a theoretical plate below the feed level of said separation column.

5. A liquid phase fluorination installation according to claim 4, in which said recovery line (11a) is located at the bottom of said separation column (2).

6. A liquid phase fluorination installation according to claim 4, in which said recovery line (11b) is located at a level comprised between that of a theoretical plate above the level of the bottom of said column and that of a theoretical plate below the feed level of said separation column (2).

7. A liquid phase fluorination installation according to any one of claims 4 to 6, in which said collection line (10) providing for recycling of said heavy fraction collected at the bottom of said separation column (2) to said fluorination reactor (1) comprises a dipleg.

## Patentansprüche

1. Verfahren zur Fluorierung in flüssiger Phase in Gegenwart eines Katalysators, das aus den folgenden Verfahrensschritten besteht: Reaktion von Fluorwasserstoffsäure und einen, organischen Ausgangsmaterial in einer Reaktionszone; Auftrennen der Reaktionsmischung in mindestens eine leichte Fraktion, die die gewünschten, fluorierten organischen Produkte und mindestens einen Teil der gebildeten, unterfluorierten organischen Produkte enthält, und eine schwere Fraktion, die unter anderem die restlichen, gebildeten, unterfluorierten organischen Produkte enthält, in einer Trennungszone; teilweise Kondensation der zuvor genannten leichten Fraktion, uni eine Gasphase, die die gewünschten, fluorierten organischen Produkte enthält, und eine flüssige Phase, die den ersten Teil der zuvor genannten, unterfluorierten organischen Produkte enthält, zu erhalten, wobei die schwere Fraktion der Reaktionszone wieder zugeführt wird und die flüssige Phase als Rückfluß dem oberen Teil der Trennungszone wieder zugeführt wird, wobei das Verfahren dadurch gekennzeichnet ist, daß die schwere Fraktion, die am Fuß der Trennungszone erhalten wird, die unter anderem die restlichen, gebildeten, unterfluorierten organischen Produkte umfaßt, außerdem einen Teil mitgeschleppten Katalysators enthält und daß eine intermediäre, dazwischenliegende Entnahme am Fuß oder nahe dem Fuß der Trennungszone bei einer Höhe durchgeführt wird, die mindestens einem theoretischen Boden oberhalb der Höhe der Entnahme am Fuß und mindestens einem theoretischen Boden unterhalb der Zufuhrhöhe für die Reaktionsmischung der Trennungszone entspricht.

2. Verfahren nach Anspruch 1, dadurch gekennzeichnet, daß die dazwischenliegende Entnahme an einer schweren Fraktion durchgeführt wird, die die Trennungszone verläßt.

3. Verfahren nach Anspruch 1, dadurch gekennzeichnet, daß die dazwischenliegende Entnahme in der Zone unterhalb der Trennungszone an einer schweren Fraktion durchgeführt wird, die leicht unterschiedlich von der schweren Fraktion ist, die an, Fuß der Trennungszone entnommen wird und aus einen, theoretischen Boden stammt, der sich oberhalb des einer Entnahmehöhe am Fuß entsprechenden theoretischen Bodens und unterhalb der Zufuhrhöhe der Reaktionsmischung der Trennungszone befindet.

4. Vorrichtung zur Fluorierung in flüssiger Phase, umfassend: einen Fluorierungsreaktor (1), dem Fluorwasserstoffsäure, eine organische Charge und ein katalytisches System zugeführt wird; eine Leitung (5) am Kopf des Reaktors, die eine Trenn(ungs)kolonne (2) speist; einen Kondensator (3), der am Kopf der Kolonne (2) befindlich ist, über eine Leitung (6) gespeist wird und eine gasförmige Fraktion, die über die Leitung (8) aufgefangen wird, sowie eine flüssige Fraktion, die über die Leitung (9) wieder dem Kopf der Kolonne (2) zugeführt wird, liefert, um einen Rückfluß dieser Fraktion zu gewährleisten; eine Sammelleitung (10), die sich am Fuß der Kolonne (2) befindet und die schwere Fraktion am Fuß der Kolonne (2) auffängt, um diese am Fuß des Reaktors (1) zu recyclieren; und eine Entnahmeleitung (11a, 11b), die an der Sammelleitung (10) oder bei einer dazwischenliegenden Höhe angeordnet ist, die sich zwischen einem theoretischen Boden oberhalb der Höhe des Fußes und einem theoretischen Boden unterhalb der Zufuhrhöhe für diese Kolonne befindet.

5. Vorrichtung zur Fluorierung in flüssiger Phase nach Anspruch 4, wobei die Entnahmeleitung (11a) am Fuß der Trennkolonne (2) angeordnet ist.

6. Vorrichtung zur Fluorierung in flüssiger Phase nach Anspruch 4, wobei die Entnahmeleitung (11b) bei einer Höhe zwischen einem theoretischen Boden oberhalb der Höhe des Fußes und einem theoretischen Boden unterhalb der Zuführhöhe der Trennkolonne (2) angeordnet ist.

7. Vorrichtung zur Fluorierung in flüssiger Phase nach einem der Ansprüche 4 bis 6, wobei die Leitung (10), die das Recyclieren der am Fuß der Trennkolonne (2) aufgefangenen schweren Fraktion zu dem Fluorierungsreaktor (1) hin sicherstellt, ein Tauchrohr ist.
